# EUROPÄISCHE PATENTSCHRIFT

(11) **EP 0 714 876 B1**
(45) Veröffentlichungstag und Bekanntmachung des Hinweises auf die Patenterteilung: **19.05.1999**
(21) Anmeldenummer: 95118352.4
(22) Anmeldetag: 22.11.1995
(51) Int. Cl.: C07C 17/38, C07C 19/03

(54) **Verfahren zur Abtrennung von Chlorwasserstoff**
Process for the separation of hydrogen chloride
Procédé de séparation d'acide chlorhydrique

(30) Priorität: 01.12.1994 DE 4442743
(43) Veröffentlichungstag der Anmeldung: 05.06.1996
(73) Patentinhaber: HOECHST AKTIENGESELLSCHAFT, 65929 Frankfurt am Main (DE)
(72) Erfinder: Kraus, Werner, D-65510 Hünstetten (DE); Vernaleken, Thomas, D-65719 Hofheim (DE); Schick, Wolfgang, Dr., D-65929 Frankfurt (DE); Roth, Peter, D-65817 Eppstein (DE)

(56) Entgegenhaltungen:
- FR-A- 2 081 871
- GB-A- 1 066 219
- GB-A- 1 172 009
- US-A- 2 402 978

## Beschreibung

Die Erfindung betrifft ein Verfahren zur Abtrennung von Chlorwasserstoff aus einem bei der Chlorierung von Methan entstehenden Gasgemisch durch Wäsche des gekühlten und komprimierten Gemischs mit mindestens einem flüssigen Chlormethan.

Es ist aus DE-PS 1 568 575 (entsprechend CA-PS 808 699) bekannt, aus der bei dieser Wäsche entstehenden flüssigen Phase, die den bei der Reaktion entstandenen Chlorwasserstoff und die Chlorierungsprodukte des Methans enthält, durch ein- oder mehrstufige Rektifikation trockenen Chlorwasserstoff abzutrennen.

Überraschenderweise wurde nun gefunden, daß man den Chlorwasserstoff viel einfacher durch Desorption aus der flüssigen Phase abtrennen kann. Die von Chlorwasserstoff befreite flüssige Phase kann anschließend, falls gewünscht, durch Destillation in die einzelnen Chlormethane aufgetrennt werden.

Gegenstand der Erfindung ist ein Verfahren zur Abtrennung von Chlorwasserstoff aus einem bei der Chlorierung von Methan entstehenden Gasgemisch, das unumgesetztes Methan, dessen Chlorierungsprodukte und bei der Reaktion entstandenen Chlorwasserstoff enthält, durch Kühlung und Kompression des Gasgemischs und anschließende Wäsche mit einer Waschflüssigkeit, die aus mindestens einem flüssigen Chlorierungsprodukt des Methans besteht, wodurch das Gasgemisch in eine Methan enthaltende Gasphase und eine Chlorwasserstoff und Chlorierungsprodukte des Methans enthaltende Flüssigphase getrennt wird, dadurch gekennzeichnet, daß man den Chlorwasserstoff durch Erwärmen aus der Flüssigphase desorbiert.

Vor der Wäsche wird der den Chlorierungsreaktor verlassende heiße Gasstrom abgekühlt, und zwar vorzugsweise auf etwa 20 bis 50°C. Anschließend wird das Gas im allgemeinen getrocknet, beispielsweise mit Hilfe eines Molsiebes. Danach wird das Gas komprimiert, vorzugsweise auf einen Druck von 7 bis 15 bar absolut. Anschließend wird es erneut auf etwa 20 bis 50°C abgekühlt.

Die darauf folgende Wäsche des Gasgemischs, bei der die Chlorierungsprodukte des Methans und der bei der Chlorierung entstandene Chlorwasserstoff gemeinsam in der Waschflüssigkeit absorbiert werden, wird im allgemeinen nahezu isotherm durchgeführt, vorzugsweise bei einem Druck von 1 bis 35 bar absolut und einer Temperatur von -50 bis +50°C, insbesondere einem Druck von 7 bis 15 bar absolut und einer Temperatur von 5 bis 15°C. Diese Wäsche wird in einer Absorptionskolonne durchgeführt, wobei die Waschflüssigkeit (ein oder mehrere flüssige Chlormethane) am Kopf aufgegeben wird, während das Gasgemisch im Gegenstrom hierzu von unten nach oben die Packungsschicht der Absorptionskolonne durchströmt.
Die bei der Wäsche nicht absorbierten Bestandteile des Gasgemischs, d.h. Methan, restlicher Chlorwasserstoff, restliche Chlormethane, Stickstoff, können als Kreisgas zum Reaktor zurückgeführt werden.
Im unteren Teil der Absorptionskolonne wird die entstandene Absorptionswärme vorzugsweise durch einen Umpumpkühler bei einer Temperatur von -20 bis +10°C abgeführt.

Die in der Absorptionskolonne mit Chlorwasserstoff und den Chlorierungsprodukten des Methans beladene Waschflüssigkeit wird mittels einer Pumpe zu einem Desorber geleitet. Anschließend wird der Chlorwasserstoff alleine desorbiert, während die Chlorierungsprodukte des Methans in der Waschflüssigkeit verbleiben. Der Desorber (Desorptionskolonne] wird im allgemeinen bei einem Druck von 1 bis 35 bar absolut, vorzugsweise 10 bis 20 bar absolut betrieben. Die Temperatur beträgt dabei am Kopf des Desorbers im allgemeinen -50 bis +40°C, vorzugsweise -20 bis +10°C. Die Sumpftemperatur des Desorbers beträgt im allgemeinen 80 bis 160°C, vorzugsweise 90 bis 110°C. Der Chlorwasserstoff wird über Kopf entnommen.

Am Sumpf des Desorbers werden die chlorwasserstofffreien Chlormethane abgezogen. Ein Teil davon wird wieder als Waschflüssigkeit in der Absorptionskolonne benutzt. Der Rest kann destillativ in die einzelnen Chlormethane aufgetrennt werden. Anschließend können, falls gewünscht, sowohl Monochlormethan als auch Dichlormethan zum Reaktor zurückgeführt und weiterchloriert werden. Diese Fahrweise wird dann gewählt, wenn hauptsächlich Trichlormethan hergestellt werden soll. Bei vollständiger Rückführung von CH₃Cl und CH₂Cl₂ werden lediglich HCl, CHCl₃ und CCl₄ als Produkte des Verfahrens gewonnen.

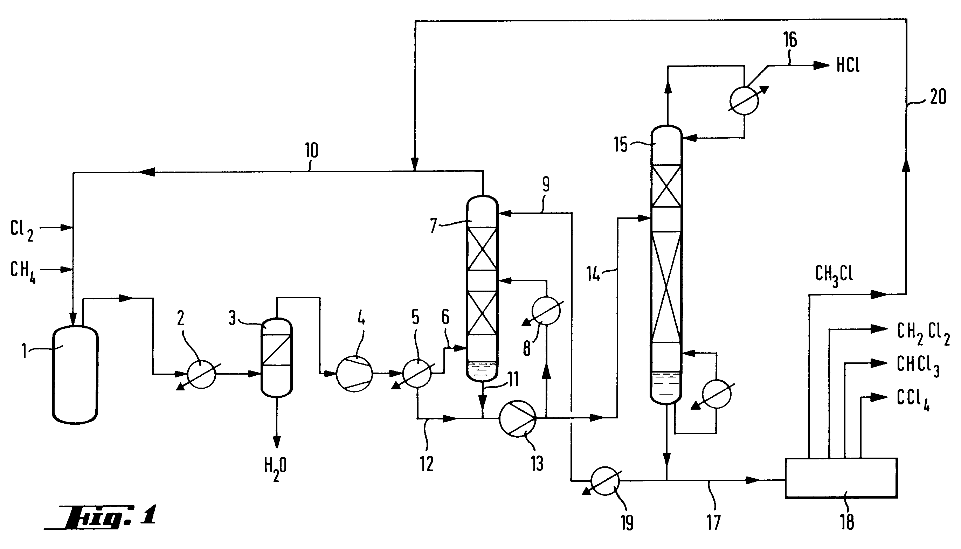

Die Erfindung soll durch das folgende Beispiel und Figur 1 näher erläutert werden.

### Beispiel

Für eine Apparatur nach Figur 1 wurde berechnet, welche Mengen an Methan, chlorierten Methanen, HCl und H₂O an den Stellen (6), (9), (10), (11), (12), (14), (16), (17) und (18) der Apparatur vorliegen würden, unter der Annahme, daß das im folgenden charakterisierte Gasgemisch im Methan-Kreislaufreaktor (1) vorliegt und entstandenes CH₃Cl vollständig zurückgeführt wird:

In dem Methan-Kreislaufreaktor (1) entsteht bei 420°C und 3,5 bar absolut folgendes Gasgemisch:

| | | |
|---|---|---|
| CH₃Cl: | 43,14 | kmol/h |
| CH₂Cl₂: | 18,08 | " |
| CHCl₃: | 12,60 | " |
| CCl₄: | 1,04 | " |
| CH₄: | 211,12 | " |
| HCl: | 371,22 | " |
| H₂O: | 0,14 | kmol/h |

Dieses Gemisch wird in Kühler (2) geleitet. Nach Abkühlung des Gases auf ca. 30°C wird es zur Abtrennung des Wassers über ein Mol-Sieb (Silikagel) in Gefäß (3) geleitet. Danach wird das Gas im Kompressor (4) auf 7 bar absolut verdichtet und anschließend in Kühler (5) erneut auf 30°C gekühlt. Der bei der Kühlung nicht kondensierte Teil des Ausgangsgases wird über Leitung (6) der Absorptionskolonne (7) zugeführt. Diese steht ebenfalls unter einem Druck von 7 bar absolut und wird mittels eines Umpumpkühlers (8) bei etwa 0°C betrieben. Am Kopf von Kolonne (7) wird als Waschflüssigkeit ein Gemisch aus Chlormethanen mit einer Temperatur von -20°C über Leitung (9) eingespeist.

Die in Kolonne (7) nicht absorbierten Bestandteile des Ausgangsgases:

| | | |
|---|---|---|
| CH₃Cl: | 23,94 | kmol/h |
| CH₂Cl₂: | 2,37 | " |
| CHCl₃: | 0,66 | " |
| CCl₄: | 0,03 | " |
| CH₄: | 206,47 | " |
| HCl: | 282,04 | " |

verlassen Kolonne (7) über Kopf und werden über Leitung (10) in Reaktor (1) zurückgeführt.

Der Sumpfablauf von Kolonne (7), bestehend aus

| | | |
|---|---|---|
| CH₃Cl: | 50,64 | kmol/h |
| CH₂Cl₂: | 37,89 | " |
| CHCl₃: | 26,57 | " |
| CCl₄: | 2,13 | " |
| CH₄: | 4,36 | " |
| HCl: | 86,16 | " |

wird über Leitung (11) entnommen und gemeinsam mit dem in Kühler (5) kondensierten und über Leitung (12) abgezogenen Teil des Ausgangsgases über Pumpe (13) und Leitung (14) dem Desorber (15) zugeführt. Hier wird am Kopf über Leitung (16) gasförmiger Chlorwasserstoff bei -20°C und 15 bar absolut abgezogen. Der chlorwasserstofffreie Sumpfablauf (ca. 100°C, 15,1 bar absolut) des Desorbers (15) wird teilweise über Leitung (17) der Destillationsanlage (18) zugeführt. Hier erfolgt die Zerlegung des Gemisches der Chlormethane in mehreren Rektifizierkolonnen zu

| | | |
|---|---|---|
| CH₃Cl: | 43,12 | kmol/h |
| CH₂Cl₂: | 18,08 | " |
| CHCl₃: | 12,60 | " |
| CCl₄: | 1,04 | " |

Der nicht der Destillation zugeführte Teil des Sumpfablaufs des Desorbers (15) wird über Kühler (19) und Leitung (9) zur Absorptionskolonne (7) geleitet und dort als Waschflüssigkeit am Kopf eingespeist.

Das CH₃Cl wird über die Leitungen (20) und (10) zum Reaktor (1) zurückgeführt.

Die folgende Tabelle zeigt die am Ausgang des Methan-Kreislaufreaktors (1) angenommenen Mengen der einzelnen Komponenten sowie die berechneten Mengen an den anderen oben genannten Stellen.

**Tabelle**

| Mengenbilanz [kmol/h] | | | | | | | | | | |
|---|---|---|---|---|---|---|---|---|---|---|
| | Ausgang von (1) | (6) | (9) | (10) | (11) | (12) | (14) | (16) | (17) | Ausgang von (18) |
| CH₃Cl | 43,14 | 40,41 | 34,17 | 23,94 | 50,64 | 2,73 | 53,37 | 0,33 | 18,87 | 43,12 |
| CH₂Cl₂ | 18,08 | 11,81 | 28,45 | 2,37 | 37,89 | 6,27 | 44,16 | - | 15,71 | 18,08 |
| CHCl₃ | 12,60 | 5,64 | 21,59 | 0,66 | 26,57 | 6,96 | 33,53 | - | 11,94 | 12,60 |
| CCl₄ | 1,04 | 0,34 | 1,82 | 0,03 | 2,13 | 0,70 | 2,83 | - | 1,01 | 1,04 |
| CH₄ | 211,12 | 210,83 | - | 206,47 | 4,36 | 0,29 | 4,65 | 4,65 | - | - |
| HCl | 371,72 | 368,20 | - | 282,04 | 86,16 | 3,02 | 89,18 | 89,18 | - | - |
| H₂O | 0,14 | - | - | - | - | - | - | - | - | - |

## Patentansprüche

1. Verfahren zur Abtrennung von Chlorwasserstoff aus einem bei der Chlorierung von Methan entstehenden Gasgemisch, das unumgesetztes Methan, dessen Chlorierungsprodukte und bei der Reaktion entstandenen Chlorwasserstoff enthält, durch Kühlung und Kompression des Gasgemischs und anschließende Wäsche mit einer Waschflüssigkeit, die aus mindestens einem flüssigen Chlorierungsprodukt des Methans besteht, wodurch das Gasgemisch in eine Methan enthaltende Gasphase und eine Chlorwasserstoff und Chlorierungsprodukte des Methans enthaltende Flüssigphase getrennt wird, dadurch gekennzeichnet, daß man den Chlorwasserstoff durch Erwärmen aus der Flüssigphase desorbiert.

2. Verfahren nach Anspruch 1, dadurch gekennzeichnet, daß man das Gasgemisch vor der Wäsche auf 20 bis 50°C abkühlt.

3. Verfahren nach Anspruch 1 oder 2, dadurch gekennzeichnet, daß man das Gasgemisch vor der Wäsche trocknet.

4. Verfahren nach einem der Ansprüche 1 bis 3, dadurch gekennzeichnet, daß man das Gasgemisch vor der Wäsche auf einen Druck von 7 bis 15 bar absolut komprimiert.

5. Verfahren nach einem der Ansprüche 1 bis 4, dadurch gekennzeichnet, daß man die Wäsche bei einem Druck von 1 bis 35 bar absolut und einer Temperatur von -50 bis +50°C durchführt.

6. Verfahren nach einem der Ansprüche 1 bis 4, dadurch gekennzeichnet, daß man die Wäsche bei einem Druck von 7 bis 15 bar absolut und einer Temperatur von 5 bis 15°C durchführt.

7. Verfahren nach einem der Ansprüche 1 bis 6, dadurch gekennzeichnet, daß man die Desorption des Chlorwasserstoffs bei einem Druck von 1 bis 35 bar absolut und einer Temperatur von -50 bis +40°C am Kopf des Desorbers, sowie einer Temperatur von 80 bis 160°C am Sumpf des Desorbers vornimmt.

8. Verfahren nach einem der Ansprüche 1 bis 6, dadurch gekennzeichnet, daß man die Desorption des Chlorwasserstoffs bei einem Druck von 10 bis 20 bar absolut und einer Temperatur von -20 bis +10°C am Kopf des Desorbers, sowie einer Temperatur von 90 bis 110°C am Sumpf des Desorbers vornimmt.

## Claims

1. A process for separating out hydrogen chloride from a gas mixture forming in the chlorination of methane, which gas mixture contains unreacted methane, its chlorination products and hydrogen chloride formed in the reaction, by cooling and compression of the gas mixture and subsequent scrubbing with a scrubbing liquid which comprises at least one liquid methane chlorination product, by which means the gas mixture is separated into a methane-containing gas phase and a liquid phase containing hydrogen chloride and methane chlorination products, which comprises desorbing the hydrogen chloride from the liquid phase by heating.

2. The process as claimed in claim 1, wherein the gas mixture is cooled to 20 to 50°C before the scrubbing.

3. The process as claimed in claim 1 or 2, wherein the gas mixture is dried before the scrubbing.

4. The process as claimed in one of claims 1 to 3, wherein the gas mixture is compressed before the scrubbing to a pressure of 7 to 15 bar absolute.

5. The process as claimed in one of claims 1 to 4, wherein the scrubbing is carried out at a pressure of 1 to 35 bar absolute and at a temperature of -50 to +50°C.

6. The process as claimed in one of claims 1 to 4, wherein the scrubbing is carried out at a pressure of 7 to 15 bar absolute and at a temperature of 5 to 15°C.

7. The process as claimed in one of claims 1 to 6, wherein the hydrogen chloride desorption is performed at a pressure of 1 to 35 bar absolute and at a temperature of -50 to +40°C at the top of the desorber and at a temperature of 80 to 160°C at the bottom of the desorber.

8. The process as claimed in one of claims 1 to 6, wherein the hydrogen chloride desorption is performed at a pressure of 10 to 20 bar absolute and at a temperature of -20 to +10°C at the top of the desorber and at a temperature of 90 to 110°C at the bottom of the desorber.

## Revendications

1. Procédé pour la séparation d'acide chlorhydrique à partir d'un mélange de gaz produit dans la chloration du méthane, qui contient du méthane n'ayant pas réagi, ses produits de chloration et de l'acide chlorhydrique formé lors de la réaction, par refroidissement et compression du mélange de gaz et lavage subséquent avec un liquide de lavage qui est constitué d'au moins un produit liquide de chloration du méthane, ce par quoi le mélange de gaz est scindé en une phase gazeuse contenant du méthane et une phase liquide contenant de l'acide chlorhydrique et des produits de chloration du méthane, caractérisé en ce que l'acide chlorhydrique est désorbé de la phase liquide par chauffage.

2. Procédé selon la revendication 1, caractérisé en ce que, avant le lavage, on refroidit à 20 - 50°C le mélange de gaz.

3. Procédé selon la revendication 1 ou 2, caractérisé en ce que, avant le lavage, on sèche le mélange de gaz.

4. Procédé selon l'une des revendications 1 à 3, caractérisé en ce que, avant le lavage, on comprime le mélange de gaz à une pression absolue de 7 à 15 bars.

5. Procédé selon l'une des revendications 1 à 4, caractérisé en ce que l'on effectue le lavage sous une pression absolue de 1 à 35 bars et à une température de -50 à +50°C.

6. Procédé selon l'une des revendications 1 à 4, caractérisé en ce que l'on effectue le lavage sous une pression absolue de 7 à 15 bars et à une température de 5 à 15°C.

7. Procédé selon l'une des revendications 1 à 6, caractérisé en ce que l'on effectue la désorption de l'acide chlorhydrique sous une pression absolue de 1 à 35 bars et à une température de -50 à +40°C en tête du désorbeur, ainsi qu'à une température de 80 à 160°C au pied du désorbeur.

8. Procédé selon l'une des revendications 1 à 6, caractérisé en ce que l'on effectue la désorption de l'acide chlorhydrique sous une pression absolue de 10 à 20 bars et à une température de -20 à +10°C en tête du désorbeur, ainsi qu'à une température de 90 à 110°C au pied du désorbeur.
